# EUROPEAN PATENT APPLICATION

(11) **EP 2 796 149 A1**
(43) Date of publication of application: **29.10.2014**
(21) Application number: 12858836.5
(22) Date of filing: 20.12.2012
(51) Int. Cl.: A61K 47/34, A61K 8/89, A61K 8/891, A61K 9/06, A61K 9/08, A61K 47/24, A61P 17/00, A61Q 19/00

(54) **COMPOSITION FOR SKIN CONTAINING SILICONE BASE**

(30) Priority: 21.12.2011 JP 2011279249
(71) Applicant: Maruho Co., Ltd., Osaka 531-0071 (JP)
(72) Inventor: HORISAWA, Eijiro, Kyoto-shi Kyoto 600-8815 (JP); IHARA, Mikito, Kyoto-shi Kyoto 600-8815 (JP)
(74) Representative: Pfenning, Meinig & Partner GbR
(86) International application number: PCT/JP2012/083063
(87) International publication number: WO 2013/094683

(57) **Abstract**

It is an object to provide a topical composition that is excellent in texture and feeling upon use. The present invention is a topical composition without water, satisfying the following: (A) the composition includes a silicone base in a proportion of 80% by weight or more; (B) the silicone base includes (b1) a silicone elastomer, (b2) a cyclic volatile methylsiloxane having 3 to 6 silicon atoms, and (b3) a linear dimethylpolysiloxane and/or linear methylphenylpolysiloxane; (C) the composition includes therein an active ingredient present in a dispersed or dissolved type; and (D) the composition does not contain any non-silicone thickener.

## Description

### TECHNICAL FIELD

The present invention relates to a topical composition (a composition for the skin) that contains a silicone base. The present invention relates more specifically to a topical composition without water that contains a silicone base and contains an active pharmaceutical ingredient in a dispersed or dissolved state.

### BACKGROUND ART

Hitherto, compositions in various dosage forms have been used as topical products for skin use. For topical compositions, the following is required: the effect of their active pharmaceutical ingredient (active ingredient) is high, that is, the active pharmaceutical ingredient is excellent in stability and transdermal permeability. As another important property, the compositions are required to be excellent in texture and feeling upon use when applied to the skin. In particular, ointments are excellent in covering performance, but are sticky, so as to be required to have such a light and smooth texture (feeling) that a sticky feeling is restrained.

As a composition excellent in texture and feeling upon use, for example, Patent Document 1 discloses a pharmaceutical composition without water having a combination of at least one active ingredient with a silicone agent including at least one organopolysiloxane elastomer having no hydrophilic group, this active ingredient being dissolved in the composition. According to Patent Document 1, this composition can give a silky touch while the composition does not have a sticky, oily or shiny effect.

The composition described in Patent Document 1 includes an active ingredient as a dissolved type. Since the active ingredient is not dissolved in the silicone agent, an aqueous solvent (for example, an alcohol type solvent such as anhydrous ethanol) is used as a solvent for dissolving the active ingredient, and liquid droplets of the aqueous solvent, in which the active ingredient is dissolved, are dispersed in the silicone agent, thereby preparing a composition of a medicament-dissolved type.

In order to disperse the aqueous solvent into the silicone agent, it is essential to use a non-silicone thickener (thickener having no silicon). However, the addition of the thickener causes deterioration in the feeling when the composition is applied onto the skin.

The composition described in Patent Document 1 contains the silicone agent in only a proportion of about 70% by weight and contains other components in a large proportion. Thus, there is a problem that an excellent texture and feeling upon use, which are properties of the silicone agent, are impaired.

### PRIOR ART DOCUMENT

### PATENT DOCUMENT

Patent Document 1: JP-A-2007-530515

### SUMMARY OF THE INVENTION

### PROBLEMS TO BE SOLVED BY THE INVENTION

Accordingly, at present, it is still desired to develop a topical composition that has a more excellent texture and feeling upon use.

### MEANS FOR SOLVING THE PROBLEMS

In consideration of the above-mentioned situation, the present inventors have repeated investigations about a base of a composition to be used for the skin, and succeeded in obtaining a topical composition that is excellent in texture and feeling upon use by combining specific silicone agents as the base. Thus, the present invention has been completed.

The present invention, which can solve the above-mentioned problems, is a topical composition without water (an anhydrous composition for the skin), which is characterized in that:
(A) the composition includes a silicone base in a proportion of 80% by weight or more,
(B) the silicone base includes:
   (b1) a silicone elastomer,
   (b2) a cyclic volatile methylsiloxane having 3 to 6 silicon atoms, and
   (b3) a linear dimethylpolysiloxane and/or linear methylphenylpolysiloxane,
(C) the composition includes therein an active ingredient present in a dispersed or dissolved type, and
(D) the composition does not contain any non-silicone thickener.

This composition includes, as its base, the silicone elastomer (b1) that is excellent in texture and feeling upon use, and further includes, as its regulating solvents for diluting the silicone elastomer to control the viscosity thereof, the cyclic volatile methylsiloxane (b2) having 3 to 6 silicon atoms, and the linear dimethylpolysiloxane and/or linear methylphenylpolysiloxane (b3). Since the composition includes the silicone base comprising the components (b1) to (b3) in a proportion of 80% by weight or more, the composition is very excellent in texture and feeling upon use.

Moreover, the composition does not contain any non-silicone thickener, so that the texture and the feeling upon use are not impaired.

The composition preferably includes the silicone base in a proportion of 83% by weight or more. The silicone base preferably includes only the components (b1) to (b3).

When the composition includes the active ingredient in a dispersed type, the composition preferably includes the silicone base in a proportion of 93% by weight or more. The ratio by weight of the components (b1) to (b3) in the silicone base is preferably as follows: b1 : b2 : b3 is 5.5 to 11.5 : 40 to 86 : 3 to 55 (provided that b1 + b2 + b3 = 100, the same requirement being to be applied hereinafter). The ratio is more preferably as follows: b1 : b2 : b3 is 8 to 11.5 : 55 to 86 : 3 to 35.

When the composition includes the active ingredient in a dissolved type, the ratio by weight of the components (b1) to (b3) in the silicone base is preferably as follows: b1 : b2 : b3 is 10 to 11.5 : 73 to 85 : 3 to 17 (provided that b1 + b2 + b3 = 100, the same requirement being to be applied hereinafter). The ratio is more preferably as follows: b1 : b2 : b3 is 10.7 to 11.5 : 78 to 85 : 3 to 11.5.

### EFFECT OF THE INVENTION

The composition according to the present invention includes the silicone base including the three specific components, and further the mix proportion of the silicone base is high; thus, the composition is very excellent in texture onto the skin, and feeling upon use.

### MODE FOR CARRYING OUT THE INVENTION

Preferred examples of the silicone elastomer (b1) according to the present invention include a silicone elastomer produced by a silicone elastomer production method as disclosed in JP-A-11-222556 (EP 0915120; US 5929164), the method comprising the step of combining and reacting the following components (A) to (D):
(A) a ≡Si-H containing polysiloxane of formula R3SiO(R'2SiO)ₐ(R"HSiO)_{b}SiR3 and optionally a ≡Si-H containing polysiloxane of formula HR2SiO(R'2SiO)_{c}SiR2H or a =Si-H containing polysiloxane of formula
   HR2SiO(R'2SiO)ₐ(R"HSiO)_{b}SiR2H wherein R, R', and R" are alkyl groups of 1 to 6 carbon atoms; a is 0-250; b is 1-250; and c is 0-250,
(B) an alkene,
(C) a platinum group metal catalyst; and
(D) a solvent compound,
until said silicone elastomer is formed by crosslinking and addition of ≡Si-H across double bonds in said alkene. These elastomers are commercially available, and are available from, for example, Dow Corning Corp.

Examples of the cyclic volatile methylsiloxane (b2) having 3 to 6 silicon atoms according to the present invention include hexamethylcyclotrisiloxane, octamethylcyclotetrasiloxane, decamethylcyclopentasiloxane, and dodecamethylcyclohexasiloxane. Particularly preferred is decamethylcyclopentasiloxane.

The linear dimethylpolysiloxane (b3) according to the present invention may be a polysiloxane having 5 to 652 silicon atoms and a viscosity of 2 to 5000 mm²/s. Such a linear dimethylpolysiloxane is commercially available, and is available from, for example, Dow Corning Corp.

The linear methylphenylpolysiloxane (b3) according to the present invention may be a polysiloxane having 3 to 412 silicon atoms and a viscosity of 10 to 1000 mm²/s. Such a linear methylphenylpolysiloxane is commercially available, and is available from, for example, Dow Corning Corp.

The component (b3) in the present invention is more preferably linear dimethylpolysiloxane.

The silicone base according to the present invention may contain a different silicone agent besides the components (b1) to (b3). The different silicone agent may be, for example, linear methylhydrogenpolysiloxane. The agent may also be a modified polysiloxane in which an organic group is introduced into a side chain or an end (an alcohol-, alkyl-, aralkyl-, polyether-, fatty-acid-ester-, amino-, epoxy-, carboxyl- or methacryl-modified polysiloxane).

The mix proportion of the different silicone agent in the silicone base according to the present invention is preferably 10% by weight or less, more preferably 5% by weight or less, even more preferably 3% by weight or less. The silicone base according to the present invention particularly preferably includes only the components (b1) to (b3) without including any other silicone agent.

The active ingredient included in the composition according to the present invention may be hydrophilic or lipophilic (lipid-soluble). Preferred examples of the active ingredient in the composition according to the present invention include steroids, immunosuppressive substances, anticancer agents, and a substance having an activity at microdose (such as active type vitamin D₃, antibiotics, antibacterial agents, steroid type or non-steroid type anti-inflammatory agents, opioid type medicaments, natural organic compounds, and terpenes). However, the active ingredient is not limited to these ingredients. The composition according to the present invention may contain two or more of these active ingredients.

The above-mentioned active ingredient may be present in a dispersed type (medicament-dispersed type) in the composition, present in a dissolved type (medicament-dissolved type), or present in the type that the ingredient is partially dispersed while the rest thereof is dissolved (partially dispersed type) . Whether the active ingredient is present in a dispersed type or in a dissolved type can be judged on the basis of whether or not a dispersed crystal thereof is observed through a microscope.

An appropriate mix proportion of the active ingredient in the composition cannot be mentioned without reservation since the proportion varies depending on the kind of the active ingredient. In general, the proportion properly ranges from 0.001 to 5% by weight.

When the active ingredient is an active compound high in pharmacological effect (such as a steroid, an immunosuppressive substance, or a substance having an activity at microdose), the mix proportion thereof more properly ranges from 0.001 to 0.5% by weight.

The amount and the frequency of administrations of the composition according to the present invention onto the skin may be appropriately regulated in accordance with the symptom of the skin or the active ingredient in the composition.

When the composition is a composition in a medicament-dispersed type (or in a partially dispersed type), the ratio by weight of the components (b1) to (b3) in the silicone base is as follows: b1 : b2 : b3 is preferably 5.5 to 11.5 : 40 to 86 : 3 to 55, more preferably 8 to 11.5 : 55 to 86 : 3 to 35, particularly preferably 8.2 to 11.5 : 60 to 84 : 4 to 32.

When the composition is a composition in a medicament-dissolved type, the ratio by weight of the components (b1) to (b3) is as follows: b1 : b2 : b3 is preferably 10 to 11.5 : 73 to 85 : 3 to 17, more preferably 10.7 to 11.5 : 78 to 85 : 3 to 11.5, particularly preferably 10.7 to 11.5 : 78 to 84 : 4 to 11.3.

The viscosity of the composition preferably ranges from 10 to 100,000 mPas. Thus, the ratio by weight of the components (b1) to (b3) in the silicone base may be regulated to allow the composition to have a viscosity in this range. When the composition is a high-viscosity composition in a form close to a gel form, the viscosity can be measured at a measuring temperature of 25°C, a rotation number of 20 rpm and a measuring period of 30 seconds, using a B type viscometer [TVB-10H, rotor: No. 7]. When the composition is a low-viscosity composition in a form close to a liquid form, the viscosity can be measured at a measuring temperature of 25°C, a rotation number of 50 rpm and a measuring period of 10 seconds, using a B type viscometer [TVB-10H, rotor: HH-12]. When the composition is a composition in a medicament-dispersed type, the viscosity more preferably ranges from 100 to 50,000 mPas. When the composition is a composition in a medicament-dissolved type, the viscosity more preferably ranges from 1,000 to 50,000 mPas. As the mix proportion of the silicone elastomer (b1) is higher, the composition is higher in viscosity. As the mix proportion of the cyclic volatile methylsiloxane (b2) or the linear dimethylpolysiloxane/linear methylphenylpolysiloxane (b3) is higher, the composition is lower in viscosity. The viscosity can be finely adjusted by making an appropriate control of the ratio of the cyclic volatile methylsiloxane (b2) to the linear dimethylpolysiloxane/linear methylphenylpolysiloxane (b3).

In the case of preparing the composition in which the active ingredient (hydrophilic or lipophilic) is included in a dispersed state (medicament-dispersed type composition), the base (one obtained by removing the active ingredient from the composition) may include only the above-mentioned silicone base. Thus, the composition can be prepared without using any different base component. However, a component to be mixed into an ordinary topical composition, such as a colorant, a perfume, a pigment, an antioxidant, and an ultraviolet absorber may be used if necessary. In the case of preparing the medicament-dispersed composition, the mix proportion of the silicone base in the composition is preferably 93% by weight or more of the composition, more preferably 96% by weight or more thereof, particularly preferably 98% by weight or more thereof.

In the case of preparing the composition in which the active ingredient (lipophilic) is included in a dissolved type (medicament-dissolved composition), the lipophilic active ingredient may be dissolved in an oily solvent (also referred to as a nonaqueous solvent or oily base) and mixed with the silicone base. At this time, the oily solvent in which the active ingredient is dissolved is homogeneously miscible with the silicone base; thus, even when a surfactant or a non-silicone thickener (such as a thickener having, as its base, a pasty or solid hydrocarbon such as wax) is not used, the medicament-dissolved composition can be obtained. The use of the non-silicone thickener makes the composition heavy in feeling upon use, and the use of the surfactant makes the composition poor in safety. Even when the composition of the present invention does not include these materials, the composition can be prepared as a medicament-dissolved composition. When the medicament-dissolved composition is prepared, the mix proportion of the silicone base in the composition is preferably 85% by weight or more of the composition, more preferably 88% by weight or more thereof, particularly preferably 93% by weight or more thereof.

Examples of the oily solvent used to dissolve the lipophilic active ingredient include fatty acids, fatty acid esters, higher alcohols, and hydrocarbons. Particularly preferred examples of the oily solvent include diethyl sebacate, diisopropyl sebacate, diisopropyl adipate, triglyceride of a middle-chain fatty acid, propylene glycol fatty acid esters, glycerin triisooctanate, isostearic acid, hexyldecanol, oleyl alcohol, isostearyl alcohol, octyldodecanol, isopropyl palmitate, isopropyl myristate, cetyl 2-ethylhexanoate, octyldodecyl myristate, hexadecyl isostearate, isostearyl palmitate, oleyl oleate, purified jojoba oil, squalane, liquid paraffin, and light liquid paraffin. The proportion of the oily solvent in the composition is preferably 15% by weight or less, more preferably from 3 to 10% by weight.

The composition according to the present invention is a composition without water, and does not essentially include water. In the present specification, the phrase "the composition does not essentially include a material" means that the material is not intentionally added to the composition, and the composition includes the material only when the material is inevitably incorporated into the composition. The composition according to the present invention does not essentially include an aqueous component (such as a lower alcohol or a polyhydric alcohol), or includes the aqueous component only in a small proportion. That is, the composition according to the present invention is a composition in which the total amount of water (included only when it is inevitably incorporated into the composition; the proportion thereof is usually less than 1% by weight) and any aqueous component is 5% by weight or less of the composition, preferably 3% by weight or less thereof, more preferably 1% by weight or less thereof. The composition is particularly preferably a composition which does not include water at all, more preferably a composition which does not include water or any aqueous component at all.

A preferred pharmaceutical form of the composition according to the present invention is a pharmaceutical form exhibiting a gel form or a liquid form, for example, an oily gel form like an ointment, or a lotion.

The composition according to the present invention may contain a component to be mixed into an ordinary topical composition, such as a colorant, a perfume, a pigment, an antioxidant, and an ultraviolet absorbent if necessary. The proportion of the additive component in the composition is preferably 10% by weight or less, more preferably 3% by weight or less, particularly preferably 1% by weight or less.

Names of the preferred compounds of the essential components and the optional components used in the composition of the present invention are described in the preceding paragraphs. Examples of the external application agent of the present invention include compositions in which these compounds are arbitrarily combined, and further include compositions in which the respective concentration ranges described about the individual components are arbitrarily combined. The respective numerical ranges of the concentrations, the viscosities, and the like that are described in the preceding paragraphs may be arbitrarily combined. When plural numerical ranges are described, the respective upper limit values of the numerical ranges or the respective lower limits thereof may be arbitrarily combined.

A description is made about examples of a method for producing the composition according to the present invention. When the composition according to the present invention is in a medicament-dispersed type, the method is, for example, a method of: adding a component (b3) to a preliminary mixture of components (b1) and (b2) and stirring the resultant, or adding components (b2) and (b3) to a component (b1) and then stirring the resultant to prepare a mixture of the components (b1 to b3) ; and subsequently using a homo-mixer to disperse an active ingredient therein. When the composition according to the present invention is in a medicament-dissolved type, the method is, for example, a method of: adding a component (b3) to a preliminary mixture of components (b1) and (b2) and stirring the resultant, or adding components (b2) and (b3) to a component (b1) and then stirring the resultant to prepare a mixture of the components (b1 to b3); and subsequently mixing an active ingredient dissolved in advance in an oily solvent with the mixture while stirring.

The composition obtained by the producing process can be regulated into a form from a semi-solid form to a liquid form by regulating the mix ratio of the components (b1) to (b3). More specifically, by making the proportion of the amount of the component(s) (b2) and/or (b3) small, a semi-solid form composition like an ointment can be prepared. By making that of the component(s) (b2) and/or (b3) large, a lotion-form composition can be prepared.

### EXAMPLES

Hereinafter, the present invention will be described in more detail by way of Examples thereof. However, the present invention is not limited to Examples.

### [Example 1] Preparation and Evaluation of Medicament-Dispersed Composition

Each composition shown in Tables 1 and 2 was prepared by the following procedure. As its active ingredients, a steroid (betamethasone butyrate propionate) and active type VD₃ (maxacalcitol) were used. As a mixture of a silicone elastomer (b1) and a cyclic volatile methylsiloxane [decamethylcyclopentasiloxane] (b2), ST-Elastomer 10 (the ratio by weight of the silicone elastomer : decamethylcyclopentasiloxane = 12 : 88), manufactured by Dow Corning Corp. , was used As a linear dimethylpolysiloxane (b3), Q7-9120 Silicone Fluid 100 CST, manufactured by Dow Corning Corp., was used.

### Method for Preparing Medicament-Dispersed Composition

(1) A mixture of components (b1) and (b2) was weighed and taken into an appropriate container.
(2) A component (b3) was added to the resultant in (1), and these components were mixed.
(3) Active ingredients were added to the resultant in (2), and these components were stirred at room temperature in a homo-mixer (at 6000 rpm for 5 minutes) to disperse the active ingredients.

The compositions prepared as described above were white and semitransparent compositions that were each in a gel form or a liquid form. Each of the compositions was observed through a microscope. As a result, the dispersion of a crystal was observed, so that it was confirmed that the composition was a medicament-dispersed composition.

About each of the compositions, an appropriate amount thereof was applied onto the skin, and then the texture (feel) of the composition was evaluated in accordance with whether or not the composition was small in stickiness to have a smooth texture. Four examinees each evaluated the composition. The composition was valued at AA when extremely good in texture; at A when good therein; at B when fair therein; and at C when poor therein. The average of the four examinees' values was fixed as the value of the composition.

The compositions shown in Tables 1 and 2 showed a higher viscosity as the mix proportion of the silicone elastomer (b1) was larger. Of the compositions shown in the tables, the respective viscosities of the compositions Nos. 007, and 009 to 011 were measured under low viscosity measuring conditions (conditions 1) while the viscosity of the composition No. 002 was measured under high viscosity measuring conditions (conditions 2).

Conditions 1: B-type viscometer (TBV-10H); rotor: No. HH-12; rotation number: 50 rpm; measuring period: 10 seconds; and measuring temperature: 25°C.

Conditions 2: B-type viscometer (TBV-10H); rotor: No. 7; rotation number: 20 rpm; measuring period: 30 seconds; and measuring temperature: 25°C.

The results are shown in Table 1.

**Table 1: Medicament-dispersed composition (numerical values in Table each represent part(s) by weight)**

| | | | No. 001 | No.002 | No. 003 | No. 004 | No. 005 | No. 006 | No.007 | No. 008 | No.009 | No.010 | No.011 |
|---|---|---|---|---|---|---|---|---|---|---|---|---|---|
| Silicone base | Trade name | ST-Elastomer 10 (mixture of silicone elastomer and cyclic volatile methylsiloxane) | 100 | 95 | 90 | 85 | 80 | 75 | 70 | 60 | 50 | 40 | 30 |
| | | Q7-9120 Silicone Fluid 100 cSt (linear dimethylpolysiloxane) | 0 | 5 | 10 | 15 | 20 | 25 | 30 | 40 | 50 | 60 | 70 |
| Active ingredient | Ingredient name | Betamethasone butyrate propionate | 0.05 | 0.05 | 0.05 | 0.05 | 0.05 | 0.05 | 0.05 | 0.05 | 0.05 | 0.05 | 0.05 |
| | Ingredient name | Maxacalcitol | 0.0025 | 0.0025 | 0.0025 | 0.0025 | 0.0025 | 0.0025 | 0.0025 | 0.0025 | 0.0025 | 0.0025 | 0.0025 |
| Texture (feeling) | | | C | A | AA | A | A | A | A | B | B | C | C |
| Viscosity (mPas) | | | | 35486 (Conditions 2) | | | | | 180 (Conditions 1) | | 84 (Conditions 1) | 82 (Conditions 1) | 83 (Conditions 1) |

**Table 2: Ratio by weight of respective components constituting silicone base in Table 1; and percentage by weight of silicone base in composition**

| | | | No.001 | No.002 | No.003 | No.004 | No.005 | No.006 | No.007 | No.008 | No.009 | No.010 | No.011 |
|---|---|---|---|---|---|---|---|---|---|---|---|---|---|
| Silicone base | Component name | (b1) Silicone elastomer | 12.0 | 11.4 | 10.8 | 10.2 | 9.6 | 9.0 | 8.4 | 7.2 | 6.0 | 4.8 | 3.6 |
| | | (b2) Cyclic volatile methylsiloxane | 88.0 | 83.6 | 79.2 | 74.8 | 70.4 | 66.0 | 62.6 | 52.8 | 44.0 | 35.2 | 26.4 |
| | | (b3) Linear dimethylpolysiloxane | 0.0 | 5.0 | 10.0 | 15.0 | 20.0 | 25.0 | 30.0 | 40.0 | 50.0 | 60.0 | 70.0 |
| Percentage by weight of silicone base in composition | | | 99.95 | 99.95 | 99.95 | 99.95 | 99.95 | 99.95 | 99.95 | 99.95 | 99.95 | 99.95 | 99.95 |

### [Example 2] Preparation of Medicament-Dissolved Composition

Each composition having a composition shown in Tables 3 and 4 was prepared by the following procedure. The same substances as in Example 1 were used as its active ingredients, its mixture of a silicone elastomer (b1) and a cyclic volatile methylsiloxane [decamethylcyclopentasiloxane] (b2), and its linear dimethylpolysiloxane (b3). As diethyl sebacate, NIKKOL DES-SP, manufactured by Nikko Chemicals Co., Ltd., was used.

### Method for Preparing Medicament-Dissolved Composition

(1) A mixture of components (b1) and (b2) was weighed and taken into an appropriate container.
(2) A component (b3) was added to the resultant in (1), and these components were mixed.
(3) Active ingredients dissolved in an oily solvent were added to the resultant in (2), and these components were stirred at room temperature in a homo-mixer (at 6000 rpm for 5 minutes) to mix all the components.

The compositions prepared as described above were white and semitransparent compositions that were each in a gel form or a liquid form. Each of the compositions was observed through a microscope. As a result, the dispersion of a crystal was not observed, so that it was confirmed that the composition was a medicament-dissolved composition. The compositions shown in Tables 3 and 4 showed a higher viscosity as the mix proportion of the silicone elastomer (b1) was larger. About each of these compositions, the texture (feel) thereof was evaluated in the same way as in Example 1. About the compositions Nos. 102, 105, 107, 108, and 111, the respective viscosities were measured. The compositions Nos. 105, 107, 108, and 111 were measured under the above-mentioned conditions 1 while the composition No. 102 was measured under the above-mentioned conditions 2. The results are shown in Table 3.

**Table 3: Medicament-dissolved composition (numerical values in Table each represent part(s) by weight)**

| | | | No. 101 | No.102 | No. 103 | No. 104 | No.105 | No.106 | No.107 | No.108 | No. 109 | No. 110 | No.111 |
|---|---|---|---|---|---|---|---|---|---|---|---|---|---|
| Silicone base | Trade name | ST-Elastomer 10 (mixture of silicone elastomer and cyclic volatile methylsiloxane) | 95 | 90 | 85 | 85 | 80 | 75 | 75 | 70 | 65 | 65 | 60 |
| | | Q7-9120 Silicone Fluid 100 cSt (linear dimethylpolysiloxane) | 0 | 5 | 5 | 10 | 10 | 10 | 15 | 15 | 15 | 20 | 20 |
| Oily solvent | Component name | Diethyl sebacate | 5 | 5 | 10 | 5 | 10 | 15 | 10 | 15 | 20 | 15 | 20 |
| Active ingredient | Ingredient name | Betamethasone butyrate propionate | 0.05 | 0.05 | 0.05 | 0.05 | 0.05 | 0.05 | 0.05 | 0.05 | 0.05 | 0.05 | 0.05 |
| | Ingredient name | Maxacalcitol | 0.0025 | 0.0025 | 0.0025 | 0.0025 | 0.0025 | 0.0025 | 0.0025 | 0.0025 | 0.0025 | 0.0025 | 0.0025 |
| Texture (feeling) | | | C | AA | A | A | A | B | B | C | C | C | C |
| Viscosity (mPas) | | | | 20189 (Conditions 2) | | | 1988 (Conditions 1) | | 555 (Conditions 1) | 298 (Conditions 1) | | | 85 (Conditions 1) |

**Table 4: Ratio by weight of respective components constituting silicone base in Table 3; and percentage by weight of silicone base in composition**

| | | | No. 101 | No.102 | No.103 | No.104 | No. 105 | No.106 | No.107 | No.108 | No. 109 | No.110 | No.111 |
|---|---|---|---|---|---|---|---|---|---|---|---|---|---|
| Silicone base | Component name | (b1) Silicone elastomer | 12.0 | 11.4 | 11.3 | 10.7 | 10.7 | 10.6 | 10.0 | 9.9 | 9.8 | 9.2 | 9.0 |
| | | (b2) Cyclic volatile methylsiloxane | 88.0 | 83.4 | 83.1 | 78.7 | 78.2 | 77.6 | 73.3 | 72.5 | 71.5 | 67.3 | 66.0 |
| | | (b3) Linear dimethylpolysiloxane | 0.0 | 5.2 | 5.6 | 10.6 | 11.1 | 11.8 | 16.7 | 17.6 | 18.7 | 23.5 | 25.0 |
| Percentage by weight of silicone base in composition | | | 94.95 | 94.95 | 89.95 | 94.95 | 89.95 | 84.96 | 89.95 | 84.96 | 79.96 | 84.96 | 79.96 |

It has been found from the above-mentioned Examples that according to the present invention, a composition excellent in texture can be obtained whether the composition is in a medicament-dispersed type or in a medicament-dissolved type.

## Claims

1. A topical composition without water, which is **characterized in that**:
(A) the composition comprises a silicone base in a proportion of 80% by weight or more,
(B) the silicone base comprises:
(b1) a silicone elastomer,
(b2) a cyclic volatile methylsiloxane having 3 to 6 silicon atoms, and
(b3) a linear dimethylpolysiloxane and/or linear methylphenylpolysiloxane,
(C) the composition comprises therein an active ingredient present in a dispersed or dissolved type, and
(D) the composition does not contain any non-silicone thickener.

2. The topical composition according to claim 1, comprising the silicone base in a proportion of 83% by weight or more.

3. The topical composition according to claim 1 or 2, wherein the silicone base consists of the components (b1) to (b3).

4. The topical composition according to any one of claims 1 to 3, comprising the active ingredient in a dispersed type, and comprising the silicone base in a proportion of 93% by weight or more.

5. The topical composition according to claim 4, wherein the ratio by weight of the components (b1) to (b3) in the silicone base is as follows: b1 : b2 : b3 is 5.5 to 11.5 : 40 to 86 : 3 to 55 (provided that b1 + b2 + b3 = 100).

6. The topical composition according to claim 4 or 5, wherein the ratio by weight of the components (b1) to (b3) in the silicone base is as follows: b1 : b2 : b3 is 8 to 11.5 : 55 to 86 : 3 to 35 (provided that b1 + b2 + b3 = 100).

7. The topical composition according to any one of claims 1 to 3, comprising the active ingredient in a dissolved type.

8. The topical composition according to claim 7, wherein the ratio by weight of the components (b1) to (b3) in the silicone base is as follows: b1 : b2 : b3 is 10 to 11.5 : 73 to 85 : 3 to 17 (provided that b1 + b2 + b3 = 100).

9. The topical composition according to claim 7 or 8, wherein the ratio by weight of the components (b1) to (b3) in the silicone base is as follows: b1 : b2 : b3 is 10.7 to 11.5 : 78 to 85 : 3 to 11.5 (provided that b1 + b2 + b3 = 100).
